# EUROPEAN PATENT APPLICATION

(11) **EP 1 486 165 A1**
(43) Date of publication of application: **15.12.2004**
(21) Application number: 04012651.8
(22) Date of filing: 27.05.2004
(51) Int. Cl.: A61B 5/044, A61B 5/0452, G06F 17/00

(54) **Electrocardiograph and method of displaying electrocardiographic wave**

(30) Priority: 12.06.2003 JP 2003167459
(71) Applicant: Omron Healthcare Co., Ltd., Kyoto 615-0084 (JP)
(72) Inventor: Yamamoto, Norihito, c/o Omron Healthcare Co., Ltd., Kyoto-shi Kyoto 615-0084 (JP)
(74) Representative: Kilian, Helmut, Dr.

(57) **Abstract**

An electrocardiograph and an electrocardiographic waveform display method for specifying the position of an abnormal waveform in the overall electrocardiographic waveform and displaying the specified abnormal waveform are disclosed. The overall waveform based on the electrocardiographic waveform acquired by measurement is compressed to an area EA, so that the waveform of a portion (30D) specified as an abnormality in the overall waveform is displayed in enlarged form identifiably with the abnormal waveform in the area EB of the same screen. In the overall waveform of the area EA, the position of the waveform of the portion (30D) in the area EB simultaneously displayed is designated. In the case where a plurality of partial waveforms containing an abnormal waveform exist in the overall waveform, the overall waveform can be moved along the arrow AL or AR, so that the waveform (abnormal waveform) of the portion (30D) displayed in enlarged form in the area EB can be sequentially switched to the waveform of the portion (30D) at a position in the direction of movement.

## Description

### BACKGROUND OF THE INVENTION

### 1. Field of the Invention

The present invention relates to an electrocardiograph and a method of displaying an electrocardiographic wave, and in particular to an electrocardiograph for displaying an electrocardiographic wave by a measured electrocardiographic signal and a method of displaying the electrocardiographic wave.

### 2. Description of the Related Art

The use of a portable electrocardiograph which a patient carries in daily life has extended with the increase in the cases of adult diseases caused by heart diseases.

The conventional portable electrocardiograph is divided into two types, one for making a measurement by inputting an electrocardiographic signal when a subjective symptom (event) such as palpitation or panting occurs (event type), and the other for making a measurement by comparing a measured electrocardiographic wave with a reference (resting) electrocardiographic wave stored in advance.

The portable electrocardiograph of event type conventionally proposed is configured such that heart rate trend data and event waveform data are stored together with time stamps and when the display mode is switched between trend and waveform, the same data time is maintained (the trend display range is switched between 24 hours, 60 minutes and ten minutes) (Japanese Patent Publication No. 2691814, hereinafter referred to as Reference 1). Another configuration that has been proposed is such that the heart rate trend data and the event waveform data are stored together with time stamps, and the event waveform and the trend graph are displayed simultaneously in two stages, higher and lower (time designation mark displayed/synchronous) and scrolled (Japanese Patent Publication No. 2691815, hereinafter referred to as Reference 2).

According to the conventional portable electrocardiograph of comparison type, on the other hand, a reference electrocardiographic waveform stored in advance is compared with the electrocardiographic wave detected by electrodes, and in accordance with the comparison result, the detected wave data are stored (Japanese Unexamined Patent Publication No. 9-56687, hereinafter referred to as Reference 3).

Also, a configuration has been proposed in which the normal electrocardiographic data are stored and the result of determining whether the data detected by the electrocardiographic sensor indicates arrhythmia or not, so that the arrhythmia, if any is detected, is automatically stored (Japanese Unexamined Patent Publication No. 2000-279385, hereinafter referred to as Reference 4).

Still another conventional portable electrocardiograph so far proposed has such a configuration that it is determined whether the electrocardiographic data stored in memory loop is abnormal or not, so that the abnormality frequency for each predetermined time and the electrocardiographic wave of the abnormality are stored and reproduced (Japanese Patent Publication No. 2700725, hereinafter referred to as Reference 5).

Apart from any of the types described above, a technique concerning the display has been proposed in which the display means for the electrocardiographic data stored and a transparent touch panel are integrally configured with each other, and with the display magnification of the electrocardiographic waveform and the coordinate designated by the touch key as a reference, the electrocardiograph data is displayed in enlarged or compressed form (Japanese Unexamined Patent Publication No. 5-161619, hereinafter referred to as Reference 6).

According to the measurement method of event type disclosed in References 1 and 2, the patient to whom the event waveform is presented cannot determine whether the particular waveform contains an abnormality or not.

In the measurement method of the type based on comparison with a reference waveform disclosed in Reference 3, on the other hand, no waveform determined as abnormal is visible from the electrocardiograph alone, and a diagnostic device for receiving and displaying the electrocardiographic waveform is required. Also, according to the measurement methods disclosed in References 4 and 5, the section of the waveform in the electrocardiograph data stored which has an abnormality cannot be identified. Further, in the method disclosed in Reference 6, the user cannot grasp a particular waveform contained in the stored data which is displayed in enlarged form.

### SUMMARY OF THE INVENTION

Accordingly, it is an object of this invention to provide an electrocardiograph and an electrocardiographic waveform display method capable of presenting a feature waveform such as an abnormality in the electrocardiographic wave in an easy-to-understand way.

Another object of this invention is to provide an electrocardiograph which can independently specify a feature waveform and display the specified feature waveform.

Still another object of this invention is to provide an electrocardiograph and an electrocardiographic waveform display method in which a particular portion of the electrocardiographic waveform data which provides the basis for displaying the waveform in enlarged form can be displayed identifiably.

According to one aspect of the invention, there is provided an electrocardiograph comprising a means for acquiring the electrocardiographic waveform data based on a time-series electrocardiographic signal, a display means, and a processing means for displaying the information based on the acquired electrocardiographic waveform data on the display means. The processing means includes a feature waveform specifying means for specifying the data of the portion containing a feature waveform from the acquired electrocardiographic waveform data, and a waveform display means for displaying, in different forms on a display unit, the feature waveform contained in the waveform based on the specified partial data and other waveforms.

Preferably, the feature waveform is the one indicating an abnormality of the electrocardiographic waveform.

In the case where a waveform based on the electrocardiographic waveform data is displayed, the feature waveform specified and other waveforms are displayed in different forms. Therefore, the feature waveform indicating an abnormality or the like can be identified in the electrocardiographic waveform and displayed in an easy-to-understand way.

Preferably, the waveform based on the specified partial data is displayed in enlarged form. Since the feature waveform of the partial waveform data is also displayed in enlarged form, the feature can be easily confirmed.

Preferably, the waveform display means displays the feature waveform and other waveforms at the same time. Thus, the feature waveform can be confirmed while being compared with other waveforms.

Preferably, the waveform display means displays the partial waveform based on the specified partial data after and before enlargement at the same time on the same screen. Therefore, the partial waveform and the feature waveform contained in the partial waveform before and after enlargement can be compared and checked on the same screen.

Preferably, the waveform display means displays the partial waveform and the overall waveform based on the whole electrocardiographic waveform data at the same time in different areas of the same screen, and the position of the partial waveform displayed at the same time as the overall waveform is designated in the overall waveform.

Therefore, the position of the feature waveform displayed at the same time as the overall waveform can be easily confirmed in the overall waveform.

Preferably, the waveform display means selects the partial data of the feature waveform'displayed on the display means, from a plurality of partial data containing the feature waveform specified by the feature waveform specifying means.

Once a plurality of partial data containing the feature waveform are specified in the electrocardiographic waveform data, therefore, the waveform (feature waveform) of the partial data to be displayed can be selected from the specified plurality of partial data.

Preferably, the partial data are selected in response to an instruction input from an external source. The waveform (feature waveform) of the partial data desired to be displayed, therefore, can be selected and displayed by the user, etc., inputting an instruction.

Preferably, the scale of enlargement can be set arbitrarily. Therefore, the magnification of the waveform of the partial data and the feature waveform contained therein can be changed as desired.

Preferably, the display means and the processing means are mounted in the electrocardiograph at the same time. Therefore, the feature waveform specified by the feature waveform specifying means of the processing means of the electrocardiograph can be checked through the display means of the same electrocardiograph without the intermediary of another device.

Preferably, the means for acquiring the electrocardiographic waveform data further includes an electrocardiographic measurement means for measuring the electrocardiographic waveform data by detecting an electrocardiographic signal of the human body.

Therefore, both the measurement of the electrocardiographic waveform data and the display of the information based on the measured electrocardiographic waveform data can be performed by use of the same electrocardiograph.

Preferably, the electrocardiograph further comprises a means for analyzing the feature waveform, and the data based on the analysis result are displayed on the display means. In this way, the feature waveform can be checked while at the same time confirming the analysis result data.

Preferably, the data based on the analysis result contains the comments on the necessity of diagnosis. By checking the comments, therefore, information on the seriousness of the feature waveform presented currently can be obtained as an advice, while at the same time making it possible to secure a measure for the necessity of diagnosis.

According to another aspect of the invention, there is provided an electrocardiographic waveform display method comprising the step of acquiring the electrocardiographic waveform data based on a time-series electrocardiographic signal, and the processing step for displaying the information based on the acquired electrocardiographic waveform data on the display unit prepared in advance, wherein the processing step includes the feature waveform specifying step for specifying the partial data containing the feature waveform from the electrocardiographic waveform data acquired, and the waveform display step for displaying on the display unit the feature waveform contained in the waveform based on the specified partial data and other waveforms in different forms from each other.

In displaying the waveform based on the electrocardiographic waveform data, therefore, the feature waveform specified in the particular waveform and other waveforms are displayed in different forms from each other. In this way, the feature waveform indicating an abnormality or the like can be identified in distinction with other waveforms in the electrocardiographic waveform and can be displayed in an easy-to-understand way.

### BRIEF DESCRIPTION OF THE DRAWINGS

Fig. 1 shows a functional configuration of an electrocardiograph according to an embodiment of the invention.
Fig. 2 shows an appearance of an electrocardiograph according to an embodiment of the invention.
Fig. 3 shows a diagram for explaining the measurement result data stored in the memory of Fig. 1.
Fig. 4 shows a general flowchart for the operation of an electrocardiograph according to an embodiment of the invention.
Fig. 5 shows a processing flowchart for the display mode according to an embodiment of the invention.
Fig. 6 shows an example of the display screen according to an embodiment of the invention.
Fig. 7 shows an example of the display screen according to an embodiment of the invention.
Fig. 8 shows an example of the display screen according to an embodiment of the invention.
Fig. 9 shows an example of the display screen according to an embodiment of the invention.
Figs. 10A and 10B show examples of the display screen according to an embodiment of the invention.

### DESCRIPTION OF THE PREFERRED EMBODIMENTS

Embodiments of the invention are described in detail below with reference to the accompanying drawings. In the embodiments, the display method will be explained taking a portable electrocardiograph as an example. Nevertheless, the invention is not applicable to the portable electrocardiograph alone but also to a stationary electrocardiograph with equal effect.

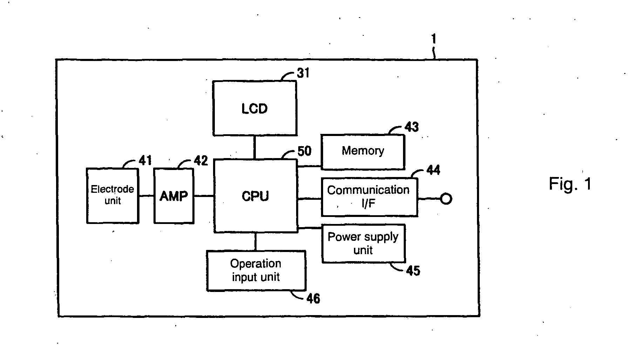

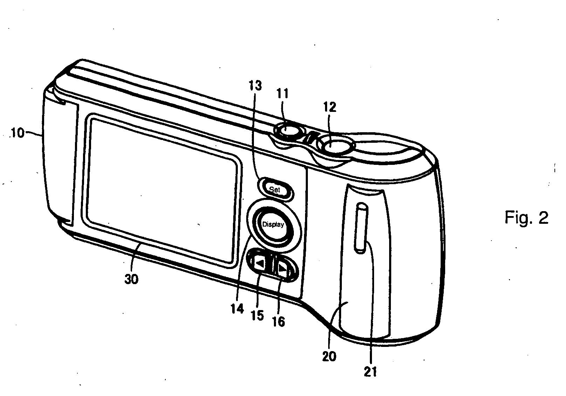

Fig. 1 shows a functional configuration of an electrocardiograph 1 according to an embodiment of the invention, and Fig. 2 an appearance of the electrocardiograph 1 of Fig. 1.

Referring to Fig. 2, the electrocardiograph 1 comprises a first electrode 10, a second electrode 20 formed with a non-slip 21 on the surface thereof, a display unit 30, a power switch 11 operated to turn on/off the power, a measurement button 12 operated to start the measurement, a setting button 13 operated to set various information (including instructions and data), a display button 14 operated to display information on the display unit 30, and scroll buttons 15, 16. The scroll button 15 is operated to move the information displayed on the display unit 30 leftward, and the scroll button 16 operated to move the information rightward.

Referring to Fig. 1, the electrocardiograph 1 comprises a CPU (central processing unit) 50 for centrally monitoring and controlling the operation of various component parts including the electrocardiographic measurement by the electrocardiograph 1, an LCD (liquid crystal display) 31 corresponding to the display unit 30 having the graphic display function, an electrode unit 41 corresponding to the first electrode 10 and the second electrode 20, an AMP (amplifier) 42 supplied with a voltage signal of the electrode unit 41 for amplifying the potential difference and outputting the potential difference to the CPU 50, a memory 43 for storing various programs and data, a communication I/F (interface) 44 for communication between the electrocardiograph 1 and other devices or an external communication network (not shown), a power supply 45 such as a battery, and an operating input unit 46 including a power switch 11, a measurement button 12, a setting button 13, a display button 14 and scroll buttons 15, 16.

At the time of measuring the electrocardiographic waveform, a measuring person such as a patient carrying the electrocardiograph 1 turns on the power switch 11, and while holding the second electrode 20 in his/her right hand using the non-slip 21 not to slip, presses the first electrode 10 against the limb and the breast other than the right hand. Under this condition, the measurement button 12 is operated. The electrocardiograph 1 switches to the measurement mode and starts the measurement of the electrocardiographic waveform. The electrocardiographic measurement session is ended within about 30 seconds. Upon lapse of about 30 seconds after starting the measurement, therefore, the measurement mode is ended automatically.

Once the electrocardiographic measurement is started, the potential signal (voltage signal) between the first electrode 10 and the second electrode 20 is input to the amplifier 42 and differentially amplified. The potential difference signal (analog signal) output from the amplifier 42 is applied to the CPU 50. The CPU 50, supplied with this signal, converts it from analog to digital signal and thereby produces electrocardiographic waveform data (digital data). The information by the electrocardiographic waveform data thus produced is displayed on the display unit 30 by the operation of the display button 14 at the time of transfer to the display mode.

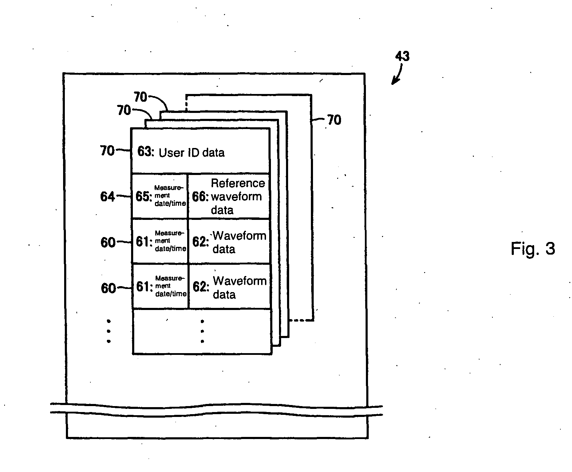

Fig. 3 shows the data representing the measurement result stored in the memory 43 of Fig. 1.

The electrocardiograph 1 according to this embodiment can be shared by a plurality of users, and a group of data 70 corresponding to each user are stored in the memory 43. The data group 70 includes measurement data 60 indicating the measurement result of the electrocardiographic waveform at the time of generation of at least one event, user identification data 63 indicating the name, etc. for identifying the corresponding user, and measurement data 64 indicating the measurement result of the reference (resting) electrocardiographic waveform.

The measurement data 64 indicate that of the resting (reference) electrocardiographic waveform not accompanied by an event, and include the measurement date/time data 65 and the reference electrocardiographic waveform data 66 obtained by measurement for about 30 seconds in the resting measurement mode.

The measurement data 60 indicate the data measured at the time of generation of an event, and includes the measurement date/time data 61 and the electrocardiographic waveform data 62 obtained by measurement for about 30 seconds in the measurement mode as of the time of generation of the event. The electrocardiographic waveform data 62, 66 indicate the level of the electrocardiographic signal with the lapse of the measurement time for indicating the electrocardiographic waveform as a trend graph (continuous waveform). The user identification data 63 is assumed to be set (designated) by operating the setting button 13, etc.

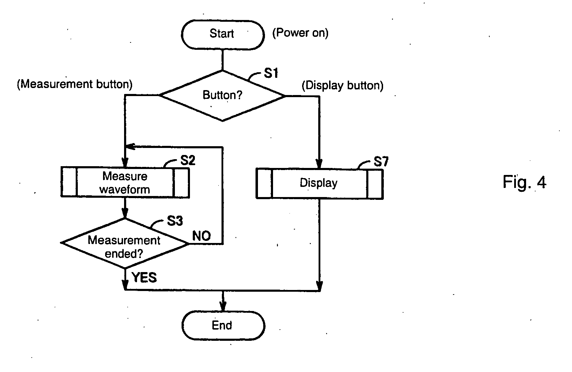

Fig. 4 shows a general flowchart for the operation in the measurement mode and the display mode of the electrocardiograph 1 according to this embodiment. With reference to Fig. 4, the electrocardiographic measurement operation of the electrocardiograph 1 is explained.

First, the user carrying the electrocardiograph 1 turns on the power switch 11, and while pressing the first electrode 10 against the limbs and the chest other than the right hand and holding the second electrode 20 by the right hand, turns on the measurement button 12 when settled in position (step S1, in which S means "step" and the word "step" may hereinafter be omitted). As a result, the operation transfers to the measurement mode and the electrocardiographic waveform is measured (S2). The measurement of the electrocardiographic waveform follows the well-known process. Briefly, the difference of the voltage signals output from the electrode unit 41 is amplified by the amplifier 42 and applied to the CPU 50. The CPU 50 converts, from analog to digital signals, the potential difference signals sequentially input thereto and detects the electrocardiographic waveform data (digital data). The electrocardiographic waveform data (the data indicating the level of the time-series electrocardiographic signals) thus detected are stored in the memory 43 in time series as the electrocardiographic waveform data 62 of the measurement data 60 for the data group 70 corresponding the user.

The CPU 50 repeatedly carries out the measurement of S2 until the detection of the lapse of 30 seconds after starting the measurement by an internal timer not shown (NO in S3). Upon the lapse of 30 seconds, the measurement end is determined (YES in S3), and the CPU 50 reads the current time data counted by the internal timer and stores the measurement date/time data 61 of the read measurement data 60. As a result, the measurement data 60 of the user are completely registered in the data group 70 corresponding to the user. After that, the series of operation for the electrocardiographic measurement is completed. Though not shown in the general flowchart of Fig. 4, the resting data are measured in the same manner as in step S2 and subsequent steps by designating the resting measurement through the operation of the setting button 13 in S1 and operating the measurement button 12. The measurement result are stored, together with the measurement date/time data 65, as the reference electrocardiographic waveform data 66 of the measurement data 64 of the data group 70 corresponding to the user in the memory 43. The resting measurement data 64 can be rewritten (changed) arbitrarily by repeating the process described above.

In the case where an event is generated and the electrocardiographic measurement is carried out for the event thus generated, an action is required urgently. Without operating the setting button 13, therefore, the measurement button 12 is turned on, and therefore the CPU 50 transfers to the measurement mode at the time of event generation.

In the measurement mode at the time of event generation, the time-series electrocardiographic waveform data for 30 seconds is similarly obtained (S2, S3). Based on this data, therefore, a new waveform data 62 corresponding to the user is detected, and additionally registered as measurement data 60 together with the waveform data 62 and the corresponding measurement date/time data 61.

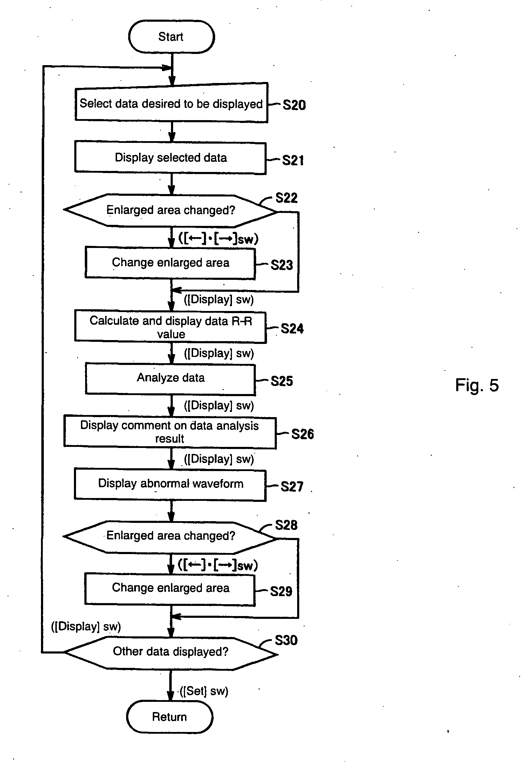

Next, the display mode associated with the steps of Fig. 5 is explained. After power is turned on, the display button 14 is turned on (S1) thereby to transfer to the display mode (S7).

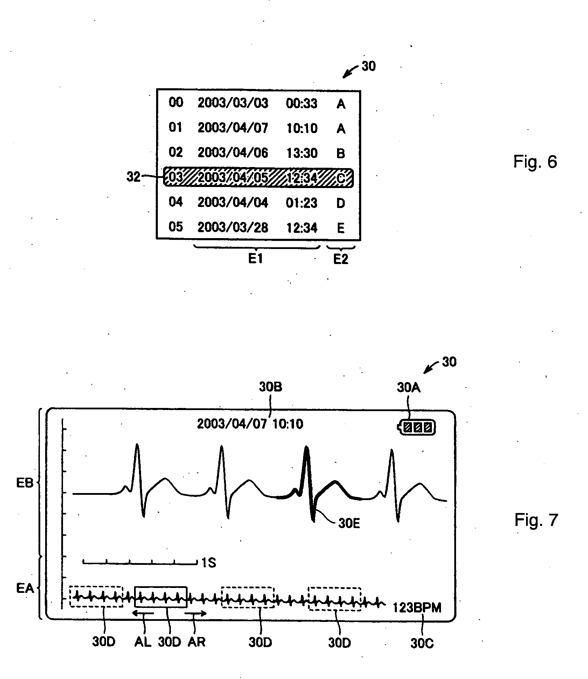

In Fig. 5, the user operates the setting button 13 in display mode and inputs a request to select the measurement data 60 desired to be displayed. In response to this request, the CPU 50 reads the data of the data group 70 for each user from the memory 43 and displays it on the display unit 30. An example of this display screen is shown in Fig. 6.

In the screen of Fig. 6, the measurement date/time data 61 of each measurement data 60 and the user identification data 63 for each user data group 70 stored in the memory 43 are displayed as a list divided into areas E1 and E2 of the screen of the display unit 30.

The user who has checked the list screen of Fig. 6 selects the measurement data 60 desired by him/her to be displayed, by operating the scroll button 15 or 16. In Fig. 6, the measurement data 60 indicated by the data 32 is selected and displayed in reverse video to notify the selection (S20).

Once the desired measurement data 60 is selected, the CPU 50 reads the selected measurement data 60 from the user data group 70, and processes and supplies the data to the display unit 30 so as to display the read measurement data 60. As a result, a screen shown in Fig. 7, for example, is displayed on the display unit 30 (S21).

The data displayed on the screen of Fig. 7 include the data 30A on the remaining capacity of the battery as the power supply unit 45, the data 30B based on the measurement date/time data 61 of the selected. measurement data 60, and the data 30C of the heart rate per minute (BPM) calculated according to well-known process based on the electrocardiographic waveform data 62 of the selected measurement data 60. Also, the screen of Fig. 7 includes an area EA and an area EB. The area EA is for displaying the whole electrocardiographic waveform, compressed as required, over the measurement time (30 seconds) based on the electrocardiographic waveform data 62 of the selected desired measurement data 60, and the area EB is for displaying, extracted in enlarged form from the whole waveform, the waveform of the portion 30D configured of a waveform having a feature (hereinafter, referred to as a feature waveform) and the surrounding waveform. The feature waveform is defined to include an abnormal waveform with an abnormal leading or trailing edge (a waveform unique to a heart disease such as arrhythmia). This abnormal waveform may be specified either by analyzing the waveform data 62 at the time of an event according to predetermined steps, or by comparing with the reference waveform data 66 in the measurement data 64 of the user. As another alternative, a waveform pattern unique to a heart disease such as arrhythmia may be registered in the memory 43 in advance, and compared to specify the abnormal waveform.

In the case where at least one portion 30D containing the feature waveform from the whole waveform is specified as described above, the CPU 50 operates in such a manner that each portion 30D specified from the overall waveform of the area EA is defined by a frame or otherwise the waveform of the particular portion 30D is displayed emphatically in highlight and presented in a way distinguishable from other waveforms. In the CPU 50, the waveform of selected one portion 30D of the specified portions 30D is enlarged and displayed in the area EB. The selected portion 30D (the second portion 30D from the left side in the area EA of Fig. 7) is displayed in a different form than the other portions 30D. In the area EB, on the other hand, the feature waveform 30E of the portion 30D is emphatically displayed (by blinking or highlighting) so that the user can identify the feature waveform.

In the case where a plurality of portions 30D are specified in the overall waveform by the CPU 50, the other portions 30D located along the arrow AL in Fig. 7 can be designated by the user operation of depressing the scroll button 15. Conversely, by operating the scroll button 16, the other portions 30D located along the arrow AR can be selectively designated (S21 to S23). Each time the portion 30D is selected by switching the designation, the form of display of the selected portion 30D in the area EA is switched to a different form than the other portions 30D, and the particular waveform is displayed in enlarged form in the area EB. At the same time, in the partial waveform displayed in enlarged form, the feature waveform 30E is displayed (emphatically) in a way distinguishable from the other waveforms (surrounding waveforms).

The scale on the ordinate (µV, mV) and the abscissa (time in sec) for the waveform in the areas EB and EA, i.e. the magnification for enlargement or compression of the waveform is determined by the calculation in the CPU 50 each time of display. The resulting waveform is displayed in accordance with the scale thus determined. The scale may alternatively be determined based on the size of the screen capable of being displayed on the display unit 30 or based on the magnification desired by the user in accordance with an value input from an external source by the user.

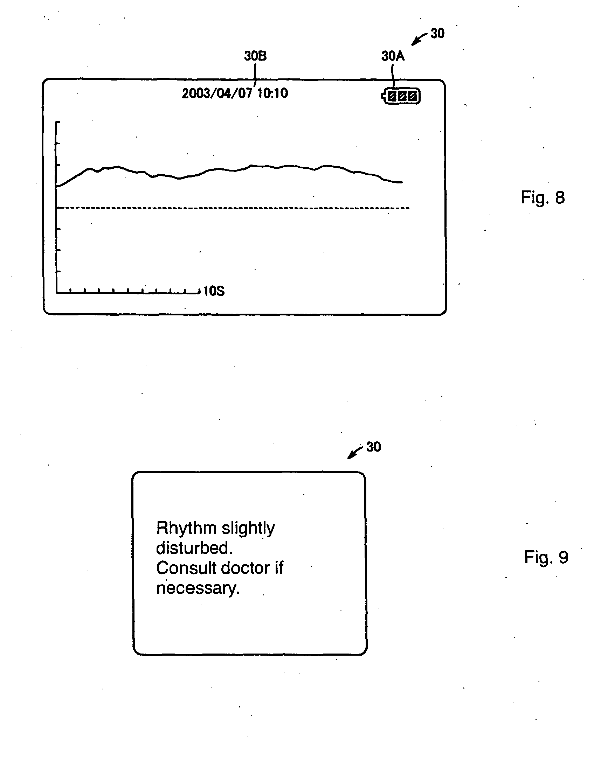

In the case where the user operates the display button 14 after the waveform of the specified portion 30D in Fig. 7 is displayed in enlarged form, the CPU 50 calculates and displays as shown in Fig. 8 the R-R value based on the electrocardiographic waveform data of the portion 30D displayed in the area EB (S24). The R-R value is calculated in the following manner. Specifically, the crest of the R wave defining one heart beat is specified by analyzing the electrocardiographic waveform data, and the heart rate is determined by retroactive calculation for time from the crest of the R wave of the immediately preceding heart beat to that of the present heart beat. The change of this heart rate is shown as a R-R value trend graph.

The user who has checked the R-R value trend graph operates the display button 14. The CPU 50 analyzes the R-R value and the electrocardiographic waveform data by a well-known method, and upon the next operation of the display button 14, displays the result of the data analysis in a comment as shown in Fig. 9 (S25, S26).

The comment is a message to the effect that the result of analysis of the electrocardiographic waveform data at the time of event generation shows the requirement to seek the advice of a specialist (doctor) or a message giving an advice that the difference from the resting waveform is so small that a medical advice should be sought as required. Normally, the measuring person cannot determine the degree of seriousness of an event simply by observing the waveform, and therefore the comment presented makes it possible to provide a measure to determine whether a medical advice should be sought or not.

When the user having checked the comment operates the display button 14, the waveform data of the portion 30D containing an abnormal waveform associated with the comment is extracted by the CPU 50 from the whole waveform data and displayed (S27).

The user, having checked the abnormal electrocardiographic waveform data displayed, operates the scroll button 15 or 16. Then, the portion 30D including the abnormal electrocardiographic waveform associated with the comment is newly switched and presented (S28, S29).

Assume that the user who has confirmed the electrocardiographic waveform operates the display button 14 desiring to display the waveform data 62 of other measurement data 60. The process returns to step S20 for repeating the process for the waveform data 62 of the other measurement data 60 in similar fashion. Upon lapse of a predetermined time length without operation of the display button 14, the display mode is ended and the process returns to that shown in Fig. 4.

### (Other display examples)

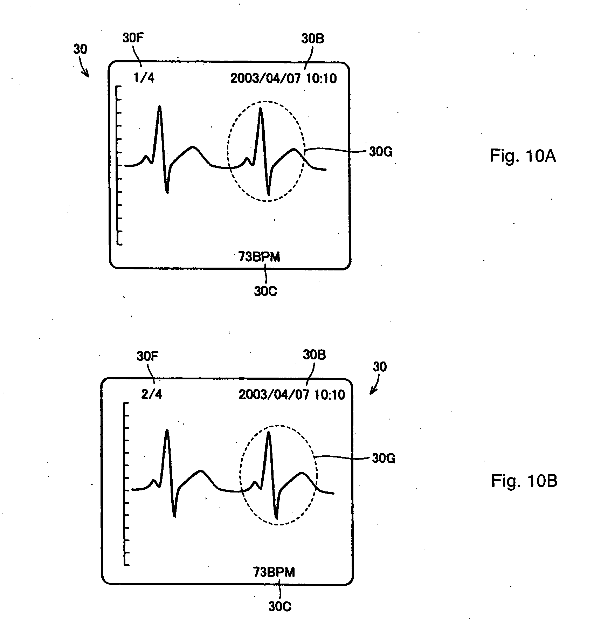

In the form of display shown in Fig. 7, a waveform enlarged from the portion 30D and a compressed overall waveform are displayed at the same time on the same screen. Nevertheless, only the enlarged waveform of the portion 30D may be displayed as shown in Figs. 10A and 10B.

The overall waveform in the area EA of Fig. 7 contains four specified portions 30D including a feature waveform. The CPU 50, instead of the display of Fig. 7, displays the feature waveform in enlarged form of the first one of the four portions 30D together with other waveforms (surrounding waveforms) as shown in Fig. 10A, the feature waveform being emphatically displayed in a different form (highlighted) than other waveforms.

The numeral "1/4" in the data 30F of the screen shown in Fig. 10A designates the feature waveform data of the first (the oldest in time series) portion 30D of the four specified portions 30D on display. Next, when the scroll button 16 is operated, as shown in Fig. 10B, the data 30F turns "2/4" so that the feature waveform data of the next (second) portion 30D is displayed. Further, assume that the scroll button 15 is operated. The waveform data of the immediately preceding portion 30D shown in Fig. 10A is displayed again.

All the embodiments disclosed above should be interpreted only as illustrative, not as limitative. The scope of the invention is defined by the claims appended hereto but not the foregoing description, and is intended to contain all the modifications without departing from the spirit and scope of the invention.

According to this invention, in the case where a waveform based on the electrocardiographic waveform data is displayed, a specified feature waveform and other waveforms in the data are displayed in different forms from each other. Therefore, a feature waveform indicating an abnormality or the like is discriminated from the other waveforms in the electrocardiographic waveform and displayed in an easy-to-understand way.

Also, an electrocardiograph itself can both specify a feature waveform and display the specified feature waveform at the same time.

Also, the data based on a particular portion of the electrocardiographic waveform data acquired can be displayed in an identifiable way in enlarged form.

## Claims

1. An electrocardiograph comprising:
a device for acquiring the electrocardiographic waveform data based on a time-series electrocardiographic signal;
a display device; and
a processing device for displaying the information based on the acquired electrocardiographic waveform data on the display device;
wherein the processing device includes a feature waveform specifying device for specifying the data of the portion containing a feature waveform from the acquired electrocardiographic waveform data, and a waveform display device for displaying on a display unit the feature waveform contained in the waveform based on the specified partial data and other waveforms, the feature waveform being displayed in different form than other waveforms

2. An electrocardiograph according to claim 1,
wherein the waveform based on the specified partial data is displayed in enlarged form.

3. An electrocardiograph according to claim 1,
wherein the waveform display device displays the feature waveform and other waveforms at the same time on the same screen.

4. An electrocardiograph according to claim 1,
wherein the waveform display device displays the enlarged partial waveform based on the specified partial data and the waveform before enlargement at the same time on the same screen.

5. An electrocardiograph according to claim 4,
wherein the waveform display device displays the partial waveform and the overall waveform based on the whole of the acquired electrocardiographic waveform data in different areas on the same screen at the same time; and
wherein the position of the partial waveform displayed at the same time as the overall waveform is designated in the overall waveform.

6. An electrocardiograph according to claim 1,
wherein the waveform display device selects the partial data of the feature waveform displayed on the display device, from a plurality of the partial data containing the feature waveform specified by the feature waveform specifying device.

7. An electrocardiograph according to claim 6,
wherein the partial data are selected in response to an instruction input from an external source.

8. An electrocardiograph according to claim 4 or 5,
wherein the scale of enlargement can be set arbitrarily.

9. An electrocardiograph according to any one of claims 1 to 8,
wherein the display device and the processing device are mounted in the electrocardiograph at the same time.

10. An electrocardiograph according to any one of claims 1 to 9, further comprising a device for analyzing the feature waveform:
wherein the data based on the result of the analysis is displayed on the display device.

11. An electrocardiograph according to claim 10,
wherein the data based on the analysis result contains a comment on the necessity of seeking a medical advice.

12. An electrocardiographic waveform display method comprising:
the step of acquiring the electrocardiographic waveform data based on the time-series electrocardiographic signal; and
the processing step for displaying the information based on the acquired electrocardiographic waveform data on the display unit prepared in advance;
wherein the processing step includes the feature waveform specifying step for specifying the partial data containing the feature waveform from the electrocardiographic waveform data acquired, and the waveform display step for displaying on the display unit the feature waveform contained in the waveform based on the specified partial data and other waveforms, the feature waveform being displayed in different form than other waveforms.
